# EUROPEAN PATENT APPLICATION

(11) **EP 4 506 032 A1**
(43) Date of publication of application: **12.02.2025**
(21) Application number: 24193357.1
(22) Date of filing: 07.08.2024
(51) Int. Cl.: A61N 1/36, G16H 20/00, G16H 50/00

(54) **AI/ML SPINAL CORD STIMULATION SIGNAL CLASSIFICATION FOR THERAPY OPTIMIZATION**

(30) Priority: 07.08.2023 US 202363531233 P; 29.07.2024 US 202418786916
(71) Applicant: Medtronic, Inc., Minneapolis, MN 55432-5604 (US)
(72) Inventor: Mueller, Jerel Keith, Saint Paul, 55117 (US); Litvak, Leonid M., 46733 Herzliya (IL); Nedrud, Joshua James, Minneapolis, 55432-3568 (US); Skerker, Abigail L., Minneapolis, 55432-3568 (US); Kharam, Aleksandra Pavlovna, Minneapolis, 55432-3568 (US); Usoro, Joshua Okon, Minneapolis, 55432-3568 (US); Brinda, Annemarie K., Minneapolis, 55432-3568 (US); Cleland, Andrew Jay, Minneapolis, 55432-3568 (US)
(74) Representative: Zimmermann & Partner Patentanwälte mbB

(57) **Abstract**

A system, device, and method support receiving a data signal from one or more sensors associated with the system in response to therapy delivered to a patient. The system, device, and method support assigning a classification to one or more portions of a waveform associated with the data signal based on characteristic information associated with the one or more portions of the waveform. The system, device, and method support providing, based on the classification, one or more parameters associated with delivering the therapy.

## Description

### CROSS-REFERENCE TO RELATED APPLICATION

This application claims the benefit of and priority to U.S. Provisional Application No. 63/531,233 filed August 7, 2023.

### FIELD OF INVENTION

The present disclosure is generally directed to therapeutic neuromodulation, and relates more particularly to classifying detected signals for therapy optimization.

### BACKGROUND

Neuromodulation therapy may be carried out by sending an electrical signal generated by a device (e.g., a pulse generator) to a stimulation target (e.g., nerves, non-neuronal cells, etc.), which may provide a desired electrophysiologic, biochemical, or genetic response in the stimulation target. Neuromodulation therapy systems may be used to deliver electrical stimulation for providing chronic pain treatment to a patient. In some neuromodulation therapies (e.g., closed-loop neuromodulation therapies), one or more signals resulting from the neuromodulation may be recorded and the therapy may be adjusted based on the recorded signals. Additionally or alternatively, the recorded signals may be used for monitoring and/or indicating conditions of the patient.

### BRIEF SUMMARY

Example aspects of the present disclosure include:
A system including: a processor; and a memory storing data thereon that, when processed by the processor, cause the processor to: receive a data signal from one or more sensors associated with the system in response to therapy delivered to a patient; assign a classification to one or more portions of a waveform associated with the data signal based on characteristic information associated with the one or more portions of the waveform; and provide, based on the classification, one or more parameters associated with delivering the therapy.

Any of the aspects herein, wherein the classification is included in a set of classifications including: a first classification indicating the one or more portions of the waveform as an electrical response by one or more anatomical elements of the patient in association with delivering the therapy; a second classification indicating the one or more portions of the waveform as a non-response by the one or more anatomical elements in association with delivering the therapy; and a third classification indicating the one or more portions of the waveform as noise.

Any of the aspects herein, wherein the one or more parameters include one or more stimulation parameters associated with delivering the therapy and evoking a response.

Any of the aspects herein, wherein the data is further executable by the processor to: provide, based on the classification, a first electrode configuration associated with delivering the therapy, a second electrode configuration associated with sensing a response to delivering the therapy, or both.

Any of the aspects herein, wherein the data is further executable by the processor to: provide at least a portion of the data signal to one or more machine learning models; and receive an output from the one or more machine learning models in response to the one or more machine learning models processing at least the portion of the data signal, wherein the output includes the classification and the one or more parameters.

Any of the aspects herein, wherein the one or more machine learning models include one or more of the following: one or more support vector machines (SVMs); one or more convolutional neural network (CNN) models; one or more feed forward neural network models; one or more transformer neural network models; and one or more decision trees.

Any of the aspects herein, wherein the waveform includes a principal component analysis (PCA) of the waveform generated based on the data signal.

Any of the aspects herein, wherein the waveform includes a raw waveform corresponding to the data signal.

Any of the aspects herein, wherein the classification indicates the one or more portions of the waveform as a non-response or noise, based on comparing the one or more portions of the waveform to one or more reference artifacts.

Any of the aspects herein, wherein the classification indicates the one or more portions of the waveform as an evoked response, based on comparing the one or more portions of the waveform to one or more waveform templates associated with a reference evoked response.

Any of the aspects herein, wherein the data signal includes an evoked compound action potential (ECAP) signal, an evoked compound muscle action potential (ECMAP) signal, or a combination thereof.

Any of the aspects herein, wherein assigning the classification is further based on at least one of: temporal information associated with the data signal; frequency information associated with the data signal; accelerometer data corresponding to one or more sensors associated with monitoring physiological information associated with the patient; impedance data corresponding to the one or more sensors; and measured values associated with the physiological information.

Any of the aspects herein, wherein assigning the classification is absent a temporal window associated with detecting the data signal by the one or more sensors.

Any of the aspects herein, wherein assigning the classification is absent a threshold value associated with the waveform.

Any of the aspects herein, wherein the classification includes an indication of at least one of: a signal type associated with the data signal; anatomical information associated with the patient and the data signal; mapping information corresponding to the one or more sensors, one or more second sensors associated with delivering the therapy, or both; the one or more parameters associated with delivering the therapy; and state information associated with the patient.

Any of the aspects herein, wherein the classification includes an indication of at least one of: predicted patient type associated with the patient; predicted pain profile information associated with the patient; and predicted device performance associated with delivering the therapy.

Any of the aspects herein, wherein the therapy includes neuromodulation therapy.

Any of the aspects herein, wherein the therapy includes at least one of: spinal cord stimulation; peripheral nerve stimulation; and pelvic stimulation.

Any of the aspects herein, wherein the data is further executable by the processor to: train a plurality of machine learning models based on a training data set associated with one or more therapies delivered to a plurality of reference patients, wherein the training data set includes a plurality of reference data signals received from one or more second sensors based on the one or more therapies delivered to the plurality of reference patients, wherein assigning the classification is based on at least one machine learning model included in the plurality of machine learning models processing at least a portion of the data signal.

Any of the aspects herein, further including a device to deliver the therapy and receive the data signal from the one or more sensors.

Any of the aspects herein, wherein the device includes at least one of a medical device, a wearable device, and an implanted device.

Any of the aspects herein, further including: a device that generates the waveform based on the data signal received from the one or more sensors.

A device including: one or more electrodes; a processor; and a memory storing data thereon that, when processed by the processor, cause the processor to: receive a data signal from the one or more electrodes in response to therapy delivered to a patient; assign a classification to one or more portions of a waveform associated with the data signal based on characteristic information associated with the one or more portions of the waveform; and provide, based on the classification, one or more parameters associated with delivering the therapy.

A method including: receiving a data signal from one or more sensors in response to therapy delivered to a patient; assigning a classification to one or more portions of a waveform associated with the data signal based on characteristic information associated with the one or more portions of the waveform; and providing, based on the classification, one or more parameters associated with delivering the therapy.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/implementations in combination with any one or more other aspects/features/implementations.

Use of any one or more of the aspects or features as disclosed herein.

It is to be appreciated that any feature described herein can be claimed in combination with any other feature(s) as described herein, regardless of whether the features come from the same described implementation.

A system, device, and method support receiving a data signal from one or more sensors associated with the system in response to therapy delivered to a patient. The system, device, and method support assigning a classification to one or more portions of a waveform associated with the data signal based on characteristic information associated with the one or more portions of the waveform. The system, device, and method support providing, based on the classification, one or more parameters associated with delivering the therapy. The details of one or more aspects of the disclosure are set forth in the accompanying drawings and the description below. Other features, objects, and advantages of the techniques described in this disclosure will be apparent from the description and drawings, and from the claims.

The preceding is a simplified summary of the disclosure to provide an understanding of some aspects of the disclosure. This summary is neither an extensive nor exhaustive overview of the disclosure and its various aspects, implementations, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, implementations, and configurations of the disclosure are possible utilizing, alone or in combination, one or more of the features set forth above or described in detail below.

Numerous additional features and advantages of the present disclosure will become apparent to those skilled in the art upon consideration of the implementation descriptions provided hereinbelow.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, implementations, and configurations of the disclosure, as illustrated by the drawings referenced below.
Fig. 1 is a diagram of a system according to at least one implementation of the present disclosure.
Fig. 2 is a diagram of a system according to at least one implementation of the present disclosure.
Fig. 3 illustrates example views of a response waveform in accordance with example aspects of the present disclosure.
Fig. 4 illustrates an example of machine learning models supported by aspects of the present disclosure.
Fig. 5 illustrates an example view of a response waveform in accordance with aspects of the present disclosure.
Fig. 6 is an example view of response waveforms in accordance with aspects of the present disclosure.
Figs. 7 and 8 illustrate example of process flows in accordance with aspects of the present disclosure.

### DETAILED DESCRIPTION

It should be understood that various aspects disclosed herein may be combined in different combinations than the combinations specifically presented in the description and accompanying drawings. It should also be understood that, depending on the example or implementation, certain acts or events of any of the processes or methods described herein may be performed in a different sequence, and/or may be added, merged, or left out altogether (e.g., all described acts or events may not be necessary to carry out the disclosed techniques according to different implementations of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any implementations of the disclosure are explained in detail, it is to be understood that the disclosure is not limited in its application to the details of construction and the arrangement of components set forth in the following description or illustrated in the drawings. The disclosure is capable of other implementations and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to the operator or user of the system, and further from the region of surgical interest in or on the patient, and distal being closer to the region of surgical interest in or on the patient, and further from the operator or user of the system.

For some neuromodulation therapies, a therapeutic electrical signal generated by a pulse generator may be sent to a stimulation target (e.g., nerves, non-neuronal cells, etc.). In a closed-loop neuromodulation therapy, a biopotential (e.g., a recorded signal) elicited with the therapeutic electrical signal may be recorded. The elicited biopotential may provide information by which to adjust the therapeutic electrical signal. Other types of closed-loop neuromodulation therapies may use and sense other types of signals to determine adjustments for the therapeutic electrical signal, such as outputs of other sensors implanted in or placed on a patient (e.g., posture sensor, accelerometer, etc.).

In some examples, spinal cord stimulation (SCS) (e.g., a form of neuromodulation that includes applying a therapeutic electrical signal or stimulation signal to nerves of the spinal cord or nerves near the spinal cord to elicit a desired electrophysiologic, biochemical, or genetic response) may be practiced in a closed-loop manner. When SCS is performed in a closed-loop manner, contacts (e.g., leads, electrodes, etc.) may be placed near a stimulation target (e.g., patient's spinal cord or a proximate structure (such as the dorsal root ganglion) or one or more targets), where the contacts are configured to apply a therapeutic electrical signal to the stimulation target to obtain a desirable electrophysiologic, biochemical, or genetic state (e.g., that leads to pain relief). For example, the therapeutic electrical signal may be configured to change how the patient's body interprets a pain signal based on causing a desired electrophysiologic, biochemical, or genetic response when applied to the stimulation target.

In some systems for providing electrical stimulation to a patient (e.g., SCS systems, systems for providing electrical stimulation for pelvic health, etc.), electrical contacts (e.g., electrodes or leads) may be used to stimulate the nerve and other contacts may be used to record an evoked response, such as the Evoked Compound Action Potential (ECAP) or the Evoked Compound Muscle Action Potential (ECMAP), resulting from the stimulation. In other closed-loop neuromodulation therapies, other signals may be recorded. For example, in deep brain stimulation, local field potentials (LFPs) may be recorded in the brain. In various types of therapies provided, the desired signal to be recorded (e.g., ECAPs, LFPs, etc.) may be recorded at a distance from the stimulation, near the stimulation, or from multiple places. In some cases, the recordings may include stimulation induced electrical artifacts, which may mask, obscure, or otherwise corrupt at least a portion of the recordings and thus, may interfere with adjusting the provided therapy.

Some techniques for providing therapy (e.g., SCS therapy) include the use of ECAPs to control the therapy. However, some algorithms leveraging ECAP signals may be highly dependent on careful setup by medical personnel. Accordingly, for example, setting up therapy parameters by medical personnel may be time consuming, ineffective, and in some cases, have varying success rates.

According to example aspects of the present disclosure, systems and techniques are disclosed which leverage the use of artificial intelligence (AI) and machine learning (ML) methods to create classification models that reduce the complexity associated with identifying an ECAP in recorded signals. The identification of ECAPs using the classification models may mitigate or eliminate the need to set up parameters (e.g., first negative peak(N1)-second positive peak(P2) windows, ECAP based therapy thresholds) associated with other algorithms for ECAP identification. In some aspects, the classification models created through the AI/ML methods described herein may support the determination of signal classifications (e.g., ECAP, No ECAP, noise) for informing other appropriate SCS applications (e.g., lead implant guidance, lead migration detection, etc.) in addition to providing therapy as described herein. In some other example implementations, rather than detection of N1-P2 windows, the systems and techniques described herein may support the determination of signal classifications (e.g., ECAP, No ECAP, noise, etc.) by detecting a correlation with a target template (e.g., an optimal template) associated with a classification. In some aspects, the systems and techniques may incorporate machine learning techniques based on which to set a filter and other correlation parameters for determining signal classifications.

Aspects of the present disclosure support implementations related to SCS sensing and using ECAPs to determine parameters (e.g., stimulation parameters, etc.) associated with dose therapy.

In an example, the systems and techniques may include trained classifiers integrated into an implantable neural stimulator (INS) or clinician programmer to identify ECAP signals. The systems and techniques support setup free classification of sensed signals for presence of an ECAP. The systems and techniques support reduced complexity associated with the setup of algorithms leveraging ECAP classifications.

According to some example implementations, the systems and techniques may support inputting acquired signals from SCS stimulation to a classifier (e.g., AI/ML trained classifiers, examples of which are described herein). The systems and techniques may support automated execution of actions and providing of notifications based on classifications (e.g., ECAP, No ECAP, noise, etc.) determined by the classifier. In some examples, the systems and techniques may support providing lead placement guidance (e.g., dorsal column, nerve root, vertebral level, etc.) in association with the classifications. Other non-limiting examples of classifications, automated actions, and notifications are later described herein.

Implementations of the present disclosure provide technical solutions to one or more of the problems of the time consuming nature and varying success rates associated with some other implementations for setting up ECAP responsive stimulation algorithms. For example, the systems and techniques described herein leveraging AI/ML methods support increased efficiency, increased success rates and effectiveness, and reduced complexity with respect to setting up stimulation algorithms, which may reduce the time spent in clinic by medical personnel and patients. In some example implementations, various AI/ML classifiers described herein may provide greater than 90% accuracy, readily determining the presence (or absence) of an ECAP signal.

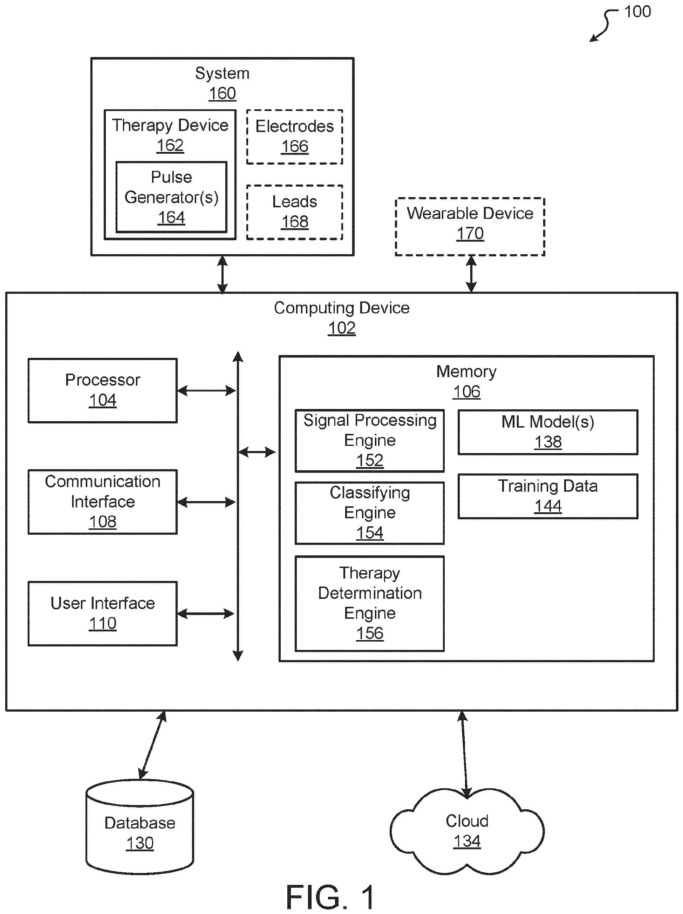

Fig. 1 illustrates an example of a system 100 that supports aspects of the present disclosure.

The system 100 includes a computing device 102, a database 130, a cloud network 134 (or other network), a system 160, and/or a wearable device 170. Systems according to other implementations of the present disclosure may include more or fewer components than the system 100. For example, the system 100 may omit and/or include additional instances of one or more components of the computing device 102, a database 130, a cloud network 134 (or other network), a system 160, and/or a wearable device 170. In an example, the system 100 may omit any instance of the computing device 102, database 130, cloud network 134 (or other network), system 160, and/or wearable device 170. The system 100 may support the implementation of one or more other aspects of one or more of the methods disclosed herein.

The computing device 102 includes a processor 104, a memory 106, a communication interface 108, and a user interface 110. Computing devices according to other implementations of the present disclosure may include more or fewer components than the computing device 102. The computing device 102 may be, for example, a control device including electronic circuitry associated with providing control signals to a therapy device 162 of the system 160.

The processor 104 of the computing device 102 may be any processor described herein or any similar processor. The processor 104 may be configured to execute instructions stored in the memory 106, which instructions may cause the processor 104 to carry out one or more computing steps utilizing or based on data received from the database 130, the cloud network 134 (or other network), the system 160, and/or the wearable device 170.

The memory 106 may be or include RAM, DRAM, SDRAM, other solid-state memory, any memory described herein, or any other tangible, non-transitory memory for storing computer-readable data and/or instructions. The memory 106 may store information or data associated with completing, for example, any step of the methods described herein, or of any other methods. The memory 106 may store, for example, instructions and/or machine learning models that support one or more functions of the computing device 102, the system 160, and/or the wearable device 170. For instance, the memory 106 may store content (e.g., instructions and/or machine learning models) that, when executed by the processor 104, enable signal processing engine 152, classifying engine 154, and/or therapy determination engine 156. Such content, if provided as in instruction, may, in some implementations, be organized into one or more applications, modules, packages, layers, or engines.

Alternatively or additionally, the memory 106 may store other types of content or data (e.g., machine learning models, artificial neural networks, deep neural networks, etc.) that can be processed by the processor 104 to carry out the various method and features described herein. Thus, although various contents of memory 106 may be described as instructions, it should be appreciated that functionality described herein can be achieved through use of instructions, algorithms, and/or machine learning models. The data, algorithms, and/or instructions may cause the processor 104 to manipulate data stored in the memory 106 and/or received from or via the computing device 102, the database 130, the cloud network 134, the system 160, and/or the wearable device 170.

The computing device 102 may also include a communication interface 108. The communication interface 108 may be used for receiving data or other information from an external source (e.g., the database 130, the cloud network 134, the system 160, the wearable device 170, and/or any other system or component separate from the system 100), and/or for transmitting instructions, data (e.g., control signals, data signals, waveforms, etc.), or other information to an external system or device (e.g., another computing device 102the database 130, the cloud network 134, the system 160, the wearable device 170, and/or any other system or component not part of the system 100). The communication interface 108 may include one or more wired interfaces (e.g., a USB port, an Ethernet port, a Firewire port) and/or one or more wireless transceivers or interfaces (configured, for example, to transmit and/or receive information via one or more wireless communication protocols such as 702.11a/b/g/n, Bluetooth, NFC, ZigBee, and so forth). In some implementations, the communication interface 108 may support communication between the therapy device 162 and one or more other processors 104 or computing devices 102, whether to reduce the time needed to accomplish a computing-intensive task or for any other reason.

The computing device 102 may also include one or more user interfaces 110. The user interface 110 may be or include a keyboard, mouse, trackball, monitor, television, screen, touchscreen, and/or any other device for receiving information from a user and/or for providing information to a user. The user interface 110 may be used, for example, to receive a user selection or other user input regarding any step of any method described herein. Notwithstanding the foregoing, any required input for any step of any method described herein may be generated automatically by the system 100 (e.g., by the processor 104 or another component of the system 100) or received by the system 100 from a source external to the system 100. In some implementations, the user interface 110 may support user modification (e.g., by a surgeon, medical personnel, a patient, etc.) of instructions to be executed by the processor 104 according to one or more implementations of the present disclosure, and/or to user modification or adjustment of a setting of other information displayed on the user interface 110 or corresponding thereto.

In some implementations, the computing device 102 may utilize a user interface 110 that is housed separately from one or more remaining components of the computing device 102. In some implementations, the user interface 110 may be located proximate one or more other components of the computing device 102, while in other implementations, the user interface 110 may be located remotely from one or more other components of the computer device 102.

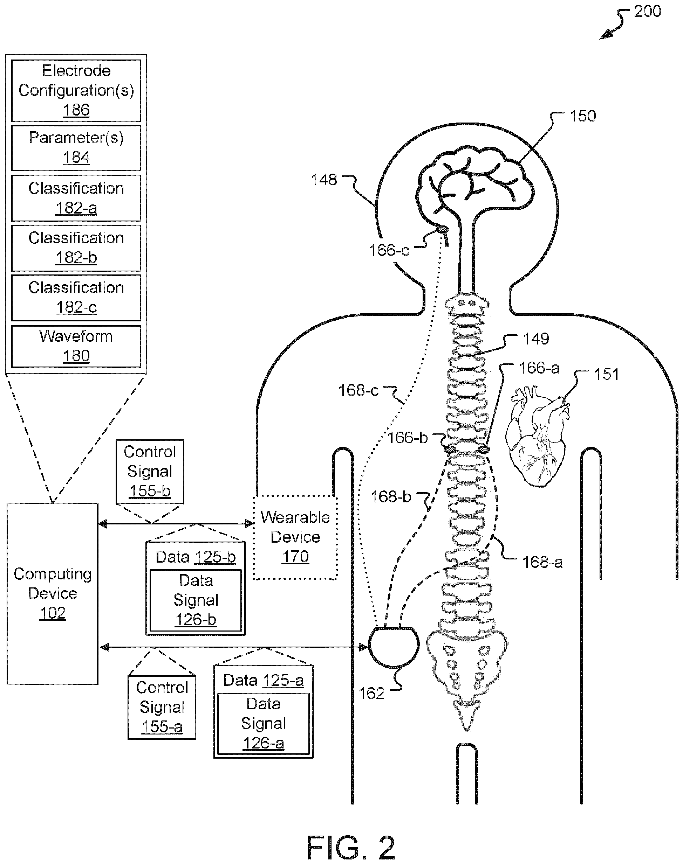

The system 160 may include a therapy device 162, electrodes 166, and leads 168. The therapy device 162 may include an implantable pulse generator (e.g., pulse generator 164). Example aspects of the system 160 and the wearable device 170 are later described herein (e.g., with reference to Fig. 2). The therapy device 162 may be configured to generate a current (e.g., therapeutic electrical signal, stimulation signal, electrical stimulation signal, etc.), and the leads 168 and the electrodes 166 may comprise a plurality of electrodes configured to carry the current from the therapy device 162 and apply the current to an anatomical element based on the electrodes being implanted on or near the anatomical element (e.g., stimulation target, for example, the spinal cord 149 of the patient 148 and/or nearby nerves to the spinal cord 149). In some examples, the therapy device 162, leads 168, and electrodes 166 may be configured to measure a physiological response of the patient 148 (e.g., prior to applying the current to the anatomical element, during application of the current, after the current is applied, etc.).

The system 160 may communicate with the computing device 102 to receive instructions for applying a current to the anatomical element and/or delivering therapy (e.g., electrical stimulation, pharmacological agent, etc.) to the anatomical element. The system 160 may also provide data (such as data received from an electrodes 166 capable of recording data), which may be used to optimize the electrodes 166 (e.g., optimize electrode placement) and/or to optimize parameters of the current generated by the therapy device 162.

Signal processing engine 152 enables the processor 104 to implement features associated with processing of data signals, waveform generation, and waveform analysis as described herein.

Classifying engine 154 may support features associated with providing and assigning classifications as described herein.

Therapy determination engine 156 enables the processor 104 to implement features associated with determining parameters (e.g., stimulation parameters associated with delivering therapy) based on a classification as described herein.

The therapy determination engine 156 enables the processor 104 to determine one or more parameters for applying the neuromodulation therapy to the anatomical element based at least in part on one or more outputs by the machine learning models 138. In an example, the therapy determination engine 156 may determine parameters or instructions that cause the therapy device 162 to employ the pulse generator 164 to generate a stimulus or that cause the therapy device 162 to deliver other therapy (e.g., a pharmacological agent, etc.). In some aspects, the therapy determination engine 156 may be referred to as a clinician programmer application.

The processor 104 may utilize data stored in memory 106 as a neural network. The neural network may include a machine learning architecture. In some aspects, the neural network may be or include one or more classifiers. In some other aspects, the neural network may be or include any machine learning network such as, for example, a deep learning network, a convolutional neural network, a reconstructive neural network, a generative adversarial neural network, or any other neural network capable of accomplishing functions of the computing device 102 described herein. Some elements stored in memory 106 may be described as or referred to as instructions or instruction sets, and some functions of the computing device 102 may be implemented using machine learning techniques.

The neural network architecture may support various inputs supportive of implementing aspects of the present disclosure. For example, the neural network architecture may support generating outputs based on model inputs including, but not limited to, record oriented data (e.g., ECAP), image data (e.g., provided by an imaging device or image sensor), and sensor data (e.g., including internet of things)).

The neural network architecture may include various appropriate model types supportive of implementing aspects of the present disclosure. For example, the neural network architecture may include statistical machine learning models (e.g., linear regression, logistic regression, decision trees, random forest, Naive bayes, ensemble methods, support vector machines, k-nearest neighbor, etc.). In some examples, the neural network architecture may include deep learning models (e.g., convolutional neural network, recurrent neural network, deep reinforcement network, deep belief network, transformer network, etc.). In some examples, the machine learning model(s) 138 may include vector machines (SVMs), convolutional neural network (CNN) models, feed forward neural network models, transformer neural network models, decision trees (e.g., random forest decision trees), and/or other machine learning models appropriate with implementing aspects of the present disclosure as described herein.

The neural network architecture may support unsupervised machine learning algorithms (e.g., principal component analysis (PCA) algorithms), semi-supervised machine learning algorithms, and supervised machine learning algorithms. The neural network architecture may support locked execution modes and continuous learning execution modes. The neural network architecture may support providing outputs including content, classifications, predictions, recommendations, and decisions.

The processor 104 may support machine learning model(s) 138 which may be trained and/or updated based on data (e.g., training data 146) provided or accessed by any of the computing device 102, the database 130, the cloud network 134, the system 160, and/or the wearable device 170. The machine learning model(s) 138 may be built and updated by the computing device 102 based on the training data 146 (also referred to herein as training data and feedback).

For example, the machine learning model(s) 138 may be trained with one or more training sets included in the training data 146. The training data 146 may include a training data set associated with one or more therapies delivered to reference patients 148. In an example, the training data 146 may include reference data signals received from sensors (e.g., electrodes 166, etc.) based on therapies delivered to the reference patients 148. In some examples, the training data 146 may include reference data signals and respective classifications corresponding to the reference data signals. In some cases, the training data 146 may include respective classifications associated with waveforms (or portions of the waveforms) that corresponding to the data signals 126.

In some aspects, the training data 146 may include multiple training sets. In an example, the training data 146 may include a first training set that includes waveform data classified as an electrical response (e.g., ECAP response, ECMAP response, etc.). In some aspects, the data included in the first training set may be associated with confirmed instances (e.g., by a healthcare provider, etc.) of the electrical response.

In another example, the training data 146 may include a second training set that includes waveform data classified as a non-response (e.g., no ECAP, etc.). In some aspects, the data included in the second training set may be associated with confirmed instances (e.g., by a healthcare provider, etc.) of the non-response. For example, the waveform data included in the second training set may include recordings which are obtained with a stimulus pulse having a signal amplitude below perception level of the subject.

In another example, the training data 146 may include a third training set that includes waveform data classified as noise. In some aspects, the data included in the third training set may include waveform data having characteristics different from waveform data associated with a response or non-response (e.g., the waveform data in the third training set may be absent characteristics associated with a response or non-response). In some aspects, the data included in the third training set may be associated with confirmed instances (e.g., by a healthcare provider, etc.) of the noise.

In some examples, based on the data included in the training data 146, the neural network may generate one or more algorithms (e.g., processing algorithms) supportive of the features described herein.

Though not shown, the system 100 may include a controller, though in some implementations the system 100 may not include the controller. The controller may be an electronic, a mechanical, or an electro-mechanical controller. The controller may comprise or may be implemented by any processor (e.g., processor 104) described herein. The controller may comprise a memory storing instructions for executing any of the functions or methods described herein as being carried out by the controller. In some implementations, the controller may be configured to simply convert signals received from the computing device 102 (e.g., via a communication interface 108) into commands for operating the system 160 (and more specifically, for actuating the therapy device 162 and the pulse generator(s) 164 thereof). In other implementations, the controller may be configured to process and/or convert signals received from the system 160 or the wearable device 170. Further, the controller may receive signals from one or more sources (e.g., system 160, wearable device 170) and may output signals to one or more sources.

The database 130 may store information that correlates one coordinate system to another (e.g., one or more robotic coordinate systems to a patient coordinate system and/or to a navigation coordinate system). The database 130 may additionally or alternatively store, for example, one or more surgical plans (including, for example, pose information about a target and/or image information about a patient's anatomy at and/or proximate the surgical site, for use by a robotic system, a navigation system, and/or a user of the computing device 102 or of the system 100); one or more images useful in connection with a surgery to be completed by or with the assistance of one or more other components of the system 100; and/or any other useful information. The database 130 may additionally or alternatively store, for example, training data 144, classification data, and the like.

The database 130 may be configured to provide any such information to the computing device 102 or to any other device of the system 100 or external to the system 100, whether directly or via the cloud network 134. In some implementations, the database 130 may include treatment information (e.g., a therapy plan) associated with a patient. In some implementations, the database 130 may be or include part of a hospital image storage system, such as a picture archiving and communication system (PACS), a health information system (HIS), and/or another system for collecting, storing, managing, and/or transmitting electronic medical records including image data.

In some aspects, the computing device 102 may communicate with a server(s) and/or a database (e.g., database 130) directly or indirectly over a communications network (e.g., the cloud network 134). The communications network may include any type of known communication medium or collection of communication media and may use any type of protocols to transport data between endpoints. The communications network may include wired communications technologies, wireless communications technologies, or any combination thereof.

Wired communications technologies may include, for example, Ethernet-based wired local area network (LAN) connections using physical transmission mediums (e.g., coaxial cable, copper cable/wire, fiber-optic cable, etc.). Wireless communications technologies may include, for example, cellular or cellular data connections and protocols (e.g., digital cellular, personal communications service (PCS), cellular digital packet data (CDPD), general packet radio service (GPRS), enhanced data rates for global system for mobile communications (GSM) evolution (EDGE), code division multiple access (CDMA), single-carrier radio transmission technology (1×RTT), evolution-data optimized (EVDO), high speed packet access (HSPA), universal mobile telecommunications service (UMTS), 3G, long term evolution (LTE), 4G, and/or 5G, etc.), Bluetooth^{®}, Bluetooth^{®} low energy, Wi-Fi, radio, satellite, infrared connections, and/or ZigBee^{®} communication protocols.

The Internet is an example of the communications network that constitutes an Internet Protocol (IP) network consisting of multiple computers, computing networks, and other communication devices located in multiple locations, and components in the communications network (e.g., computers, computing networks, communication devices) may be connected through one or more telephone systems and other means. Other examples of the communications network may include, without limitation, a standard Plain Old Telephone System (POTS), an Integrated Services Digital Network (ISDN), the Public Switched Telephone Network (PSTN), a Local Area Network (LAN), a Wide Area Network (WAN), a wireless LAN (WLAN), a Session Initiation Protocol (SIP) network, a Voice over Internet Protocol (VoIP) network, a cellular network, and any other type of packet-switched or circuit-switched network known in the art. In some cases, the communications network may include of any combination of networks or network types. In some aspects, the communications network may include any combination of communication mediums such as coaxial cable, copper cable/wire, fiber-optic cable, or antennas for communicating data (e.g., transmitting/receiving data).

The computing device 102 may be connected to the cloud network 134 via the communication interface 108, using a wired connection, a wireless connection, or both. In some implementations, the computing device 102 may communicate with the database 130 and/or an external device (e.g., a computing device) via the cloud network 134.

The system 100 or similar systems may be used, for example, to carry out one or more aspects of any of the methods described herein. The system 100 or similar systems may also be used for other purposes.

Fig. 2 illustrates an example implementation 200 of the system 100 as supported by aspects of the present disclosure. Aspects of the system 100 previously described with reference to Fig. 1 and descriptions of like elements are omitted for brevity.

The system 100, in any suitable configuration, may be used to provide a neuromodulation therapy (e.g., provide electric signals) to a patient 148 and/or carry out one or more other aspects of one or more of the methods disclosed herein. For example, the system 100 may include at least a device (e.g., computing device 102, therapy device 162, wearable device 170, etc.) that is capable of providing a stimulation applied to an anatomical element (e.g., spinal cord 149) of the patient 148 and/or to one or more nerve endings for a patient 148 (e.g., for SCS therapy).

The configuration illustrated in Fig. 2 shows application of a neuromodulation therapy and sensing of a response thereto at or near the spinal cord 149. In some examples, the therapy device 162 may include a pulse generator 164 described with reference to Fig. 1. The pulse generator 164 may be configured to generate a current or therapeutic electrical signal, such as a signal capable of stimulating a response in the spinal cord 149 or from one or more nerves. In some implementations, as described herein, the therapy device 162 may be implanted within the patient 148.

Additionally, the system 100 may include one or more leads 168 (e.g., electrical leads) that provide a connection between the therapy device 162 and the spinal cord 149 or nerves of the patient 148 for enabling, for example, stimulation. In some implementations, the leads 168 may be implanted wholly or partially within the patient 148. Additionally, or alternatively, aspects of the present disclosure support one or more leadless implementations of the system 100 for use with the peripheral nervous system (e.g., nerves that branch out from the spinal cord 149 or brain 150 of a patient 148). For example, the system 100 may support the detection of physiologic signals associated with the peripheral nervous system using sensing techniques that are absent leads 168.

In an example implementation, the therapy device 162 may be a leadless devices capable of delivering stimulation therapy without the use of a separate lead (e.g., without the use of a lead 168 for delivering stimulation therapy). For example, the therapy device 162 may be a unitary structured device that may be more robust and less invasive than lead-based counterpart devices. In some aspects, a leadless implementation of the therapy device 162 may include one or more sensors or electrodes configured to sense nerve activity or muscle activity, and provide closed loop feedback for adjustment of a stimulation therapy regime.

In some implementations, such as the one illustrated in Fig. 2, the one or more leads 168 may include a lead 168-a disposed on or connected to a first side of the spinal cord 149 of the patient 148 and a lead 168-b disposed on or connected to a second side of the spinal cord 149 of the patient 148. For example, the lead 168-a may be connected to the righthand side of the spinal cord 149, while the lead 168-b may be connected to the lefthand side of the spinal cord 149. However, the position and/or orientation of each lead relative to the spinal cord 149 may vary depending on, for example, the type of treatment, the type of lead, combinations thereof, and the like. In another example, the lead 168-a and the lead 168-b may overlap one another, and may be placed proximate one another on the dorsal side of the spinal cord 149 close to a midline of the spinal cord 149.

In some examples, the lead 168-a and the lead 168-b may both be placed on the midline of the spinal cord 149, where one of the leads 168 is cranial (e.g., anterior or nearer the head of the patient 148) and the other of the leads 168 is caudal (e.g., posterior or nearer the tail of the patient 148). Additionally or alternatively, the lead 168-a and the lead 168-b may both be placed on one side of the midline of the spinal cord 149.

Additionally, the one or more leads 168 may be connected, placed, or otherwise implanted near or on the spinal cord 149 within the patient 148, such that at least one of the one or more leads 168 are located near the heart 151 of the patient 148. For example, though not illustrated, the lead 168-a may be placed within the spinal canal behind the heart 151 (e.g., dorsally within the spinal canal, such as behind a foramen of the spine near the top of a vertebra of the thoracic vertebrae column of the spinal cord 149, or anteriorly within the spinal canal). Additionally or alternatively, as described previously, the exact placement of the one or more leads 168 may vary depending on, for example, the type of treatment, the type of lead, the patient 148, combinations thereof, and the like.

While not specifically shown in the example of Fig. 2, the one or more leads 168 may also exit the spinal cord 149 at a lumbar vertebra lower down the spinal cord 149 (e.g., the L2 vertebra, but the exact location may vary). As described herein, the one or more leads 168 being placed proximate to the heart 151 may enable the system 100 to more effectively capture signals that include cardiac activity before, during, and/or after providing a neuromodulation therapy (e.g., SCS therapy).

In other implementations, the one or more leads 168 may include at least the lead 168-a and the lead 168-b connected to other nerves of the patient 148 (e.g., the vagus nerve, different trunks of the vagus nerve, etc.). For example, the lead 168-a may be connected to a first nerve (e.g., first vagal trunk of the patient 148, such as the anterior sub diaphragmatic vagal trunk at the hepatic branching point of the vagus nerve) and the lead 168-b may be connected to a second nerve (e.g., second vagal trunk of the patient 148, such as the posterior sub diaphragmatic vagal trunk at the celiac branching point of the vagus nerve). The lead 168-a and/or the lead 168-b may be configured to provide an electrical stimulation signal from the therapy device 162 to the respective first and/or second nerve. The connection of the leads 168 to the respective nerve (or other nerves) of the patient 148 may permit the therapy device 162 to measure and/or stimulate one or more evoked potentials (e.g., ECAPs) in the patient 148 based on the provided electrical stimulation from the pulse generator 164.

Additionally, or alternatively, one lead 168 (e.g., lead 168-a) may be connected at or near the spinal cord 149 while another lead 168 (e.g., lead 168-c) may be connected at or near the brain 150 of the patient 148. Such a configuration may be used to apply a neuromodulation therapy such as a Deep Brain Stimulation (DBS). While responses to the neuromodulation therapy may be measured using one or both of the leads 168-a and 168-c, cardiac activity may also be measured using traditional medical devices, and it should be appreciated that other types of devices can be used to measure cardiac activity.

As a non-limiting example, wearable device 170 may be provided with one or more sensors that receive a data signal from the patient 148 and convert the received signal into a cardiac electrical signal (or other appropriate signal associated with measuring biometric data of the patient 148). In other words, traditional medical devices (e.g., purpose-built ECG monitors, portable ECG monitors, etc.), wearable devices 170, or any other appropriate type of device may be used to measure cardiac activity or other types of biometric data. Outputs of such device(s) may be analyzed and used to provide classifications 182, parameters 184 (e.g., stimulation parameters), and electrode configurations 186 associated with delivering therapy to patient 148 and sensing a response.

In some examples, the leads 168 may provide therapeutic electrical signals to the respective nerves via electrodes 166 or electrode devices that are connected to the nerves (e.g., sutured in place, wrapped around the nerves, etc.). In some examples, the leads 168 may be referenced as cuff electrodes or may otherwise include the cuff electrodes (e.g., at an end of the leads 168 not connected or plugged into the therapy device 162). Examples of the electrodes 166 include electrode devices, cuff electrodes, paddle electrodes, or different types of electrodes, and electrodes 166 may be disposed at a distal end of each of the leads 168.

In other examples, the leads 168 may be or comprise linear SCS leads capable of delivering one or more stimulation signals (e.g., generated by the therapy device 162) to the spinal cord 149. The leads 168 may comprise a plurality of electrodes 166 disposed along the length of the lead, such that the leads 168 contact the spinal cord 149 at multiple points along a length of the spinal cord 149. A first set of the electrodes 166 on each lead may pass an electrical signal into the spinal cord 149, while a second set of the electrodes 166 on each lead may sense one or more signals generated in response by the spinal cord 149 (e.g., recorded signals). In one or more implementations, the electrodes 166 may be able to sense, measure, or otherwise collected data related to ECAPs (e.g., ECAP waveforms). Additionally or alternatively, the electrodes 166 may be able to sense, measure, or otherwise collected data related to cardiac metrics for the patient 148 (e.g., HR, HRV, respiration, or other ECG measurements). In some examples, the therapy device 162 may be used as a contact and/or may include additional contacts for sensing, measuring, or otherwise collecting data related to biometric data (e.g., cardiac metrics) for the patient 148. A plurality of the configurations can be used to record different vectors of biometric activity towards deriving various biometric metrics.

Additionally, the system 100 may include one or more processors 104 (e.g., one or more Digital Signal Processors (DSPs), general purpose microprocessors, graphics processing units, Application Specific Integrated Circuits (ASICs), Field Programmable Gate Arrays (FPGAs), or other equivalent integrated or discrete logic circuitry) shown and described in Fig. 1 that are programmed to carry out one or more aspects of the present disclosure. In some examples, the one or more processors 104 may include a memory 106 or may be otherwise configured to perform the aspects of the present disclosure. For example, the one or more processors 104 may provide instructions to the therapy device 162, the leads 168, the electrodes 166, or other components of the system 100 not explicitly shown or described with reference to Fig. 2 for applying a neuromodulation therapy, stimulation, performing measurements (e.g., biometric measurements, cardiac metrics, ECAPs, etc.), and analyzing the same, as described herein. In some examples, the one or more processors 104 may be part of the therapy device 162 or part of a control unit for the system 100 (e.g., where the control unit is in communication with the therapy device 162 and/or other components of the system 100).

The therapy device 162 and/or wearable device 170 may be programmed to measure and record movements of the patient 148 (e.g., for the purpose of life, sleep, and activity tracking). For example, the therapy device 162 and/or wearable device 170 may comprise an accelerometer and/or other components that are designed to track and record movements of the patient 148 (e.g., whether the patient 148 is moving, not moving, laying down, standing up, running, walking, etc.). Additionally, the leads 168 and/or electrodes 166 disposed at the distal end of the leads 168 may be programmed to measure a physiological response of the patient 148.

In some examples, the physiological response may comprise an evoked response (e.g., ECAP measurement) based on applying therapy (e.g., a therapeutic electrical signal, for example, a stimulation signal) generated by the therapy device 162 to the spinal cord 149 (e.g., and/or to nearby nerves as described previously). In another example, the physiological response may include spontaneous activity (e.g., spontaneous physiological responses) by the patient 148. Additionally or alternatively, as described herein, the physiological response may comprise biometric data, for example, cardiac signals (e.g., HR, HRV, respiration, other cardiac electrogram-related measurements, etc.) of the patient 148, before, during, and after the therapeutic electrical signal is applied. In some examples, the therapy device 162 may be programmed to measure and record the biometric data (e.g., cardiac signals) via an electrode vector and/or electrodes 166 placed on an outer surface of the therapy device 162 and/or within the therapy device 162, in addition or alternative to the leads 168 and/or electrodes 166. Additionally or alternatively, an additional device (e.g., implanted within the patient 148, an external device, etc.) may be configured or programmed to record cardiac activity of the patient 148.

With reference to Figs. 1 and 2, example aspects are described that support signal classification for therapy optimization and insight in accordance with aspects of the present disclosure.

The system 100 may support the communication of data 125 (e.g., data 125-a, data 125-b, etc.) between computing device 102, therapy device 162, and wearable device 170. In an example, the system 100 may support receiving a data signal 126 (e.g., data signal 126-a) from one or more sensors (e.g., an electrode 166, an accelerometer associated with therapy device 162, a sensor integrated or coupled to wearable device 170, etc.) associated with the system 100 in response to therapy delivered to patient 148. In an example, the computing device 102 may receive data signals 126 via therapy device 162 and/or wearable device 170. Additionally, or alternatively, the computing device 102, if integrated with the therapy device 162, may receive the data signals 126 directly from the electrodes 166.

In some aspects, the data signal 126 (e.g., data signal 126-a from therapy device 162) may include an evoked compound action potential (ECAP) signal or an evoked compound muscle action potential (ECMAP) signal. In some other cases, the data signal 126 may include both an ECAP signal and an ECMAP signal. For example, the data signal 126 may include a sacral evoked response (SER) (also referred to herein as a pelvic health electrically evoked response (PEER)) which may include components of both ECAP and ECMAP.

The therapy may include neuromodulation therapy. In some example implementations, the therapy may include spinal cord stimulation (SCS), deep brain stimulation (DBS), pelvic health stimulation (e.g., associated with urinary incontinence, fecal incontinence, pelvic pain, sexual dysfunction, and other pelvic floor disorders), or other appropriate stimulation.

The system 100 may support delivery of the therapy via the therapy device 162 and/or the wearable device 170. In some aspects, computing device 102 may provide a control signal 155 to the therapy device 162 and/or the wearable device 170 in association with delivering therapy to the patient 148. In another example, the wearable device 170 may provide a control signal (not illustrated) to the therapy device 162 in association with delivering therapy to the patient 148. The example therapy described herein with reference to Fig. 2 may include delivery of electrical stimulation, but is not limited thereto. For example, the therapy may include delivery of one or more pharmacological agents.

Computing device 102 may assign a classification 182 to one or more portions of a waveform 180 associated with the data signal 126. In an example, computing device 102 may assign a classification 182 to a portion of the waveform 180 based on characteristic information associated with the portions of the waveform 180. Accordingly, for example, computing device 102 may assign classifications 182 to respective portions of the waveform 180.

In some example implementations, computing device 102 may generate the waveform 180 based on the data signal 126. In some other example implementations, therapy device 162 or wearable device 170 may generate the waveform 180 (based on the data signal 126) and provide the waveform 180 and/or the data signal 126 to the computing device 102. In some examples, the waveform 180 may be a raw waveform corresponding to the data signal 126. In another example, the waveform 180 may include a principal component analysis (PCA) of a waveform generated based on the data signal 126. Examples of the waveforms 180 (e.g., raw waveform, PCA of the waveform, etc.) are later described herein.

Computing device 102 may provide or assign classifications 182 from a set of classifications 182. In an example, the set of classifications 182 may include: a first classification 182-a (e.g., `electrical response', 'ECAP') indicating a portion of a waveform 180 as an electrical response by an anatomical element of the patient 148 in association with delivering therapy, a second classification 182-b (e.g., `non-response', `No ECAP') indicating a portion of a waveform 180 as a non-response by one or more anatomical elements in association with delivering therapy, and a third classification 182-c (e.g., 'noise') indicating a portion of a waveform 180 as noise. In some cases, the noise may be biological, electrical, or mechanical.

In some example implementations, the set of classifications 182 may include sub-classifications corresponding to any of the classifications 182. For example, for the third classification 182-c (e.g., 'noise'), computing device 102 may further provide or assign a sub-classification indicating a type (e.g., biological, electrical, mechanical, etc.) of the noise. The system 100 may apply the sub-classifications in association with diagnosing noise issues (e.g., based on noise type) in the field, which may support mitigation of noise or filtering out noise of the various types.

In an example, computing device 102 may provide or assign a classification 182-b (e.g., 'non-response') or classification 182-c (e.g., `noise') to a portion of a waveform 180 based on comparing the portion of the waveform 180 to one or more reference artifacts. For example, the reference artifacts may include reference stimulation induced electrical artifacts that correspond to a non-response or noise. In another example, computing device 102 may provide or assign a classification 182-c (e.g., `electrical response') to a portion of a waveform 180 based on comparing the portion of the waveform 180 to one or more waveform templates associated with a reference electrical response (e.g., a reference evoked response).

Other example classifications 182 (not illustrated at Fig. 2) may include an indication of a predicted patient type associated with the patient 148, predicted pain profile information associated with the patient 148, and predicted device performance (e.g., battery life) of therapy device 162. Other example aspects of the classifications 182 and data based on which the classifications 182 may be determined and assigned are later described herein.

The system 100 may support providing, based on a classification 182, one or more parameters 184 associated with delivering therapy to the patient 148. In some examples, the parameters 184 may include stimulation parameters (e.g., stimulation frequency, amplitude, duration, duty cycle, post-stimulus recharge parameters, etc.) associated with delivering therapy and evoking a response. In some other examples, the parameters 184 may include electrode configurations 186 associated with delivering therapy and sensing a response. Example aspects of the stimulation parameters and electrode configurations 186 are later described herein.

The system 100 may support providing classifications 182 (and parameters 184 associated with the classifications 182) using one or more machine learning models 138. For example, the system 100 may support providing the data signal 126 (or portion of the data signal 126) to a machine learning model 138 (e.g., implemented at the computing device 102, cloud network 134, a server, etc.). In response to the machine learning model 138 processing the data signal 126 (or at least a portion of the data signal 126), the machine learning model 138 may provide an output including a classification 182 corresponding to the data signal 126. In another example, the output may include classifications 182 respectively corresponding to portions of a waveform 180 (e.g., raw waveform) that is representative of the data signal 126. In some other examples, the output may include classifications 182 respectively corresponding to portions of a transformation (e.g., a PCA) of the waveform 180. Examples of the classifications 182 respective to portions of the waveform 180 and/or the transformation (e.g., PCA) are later described herein.

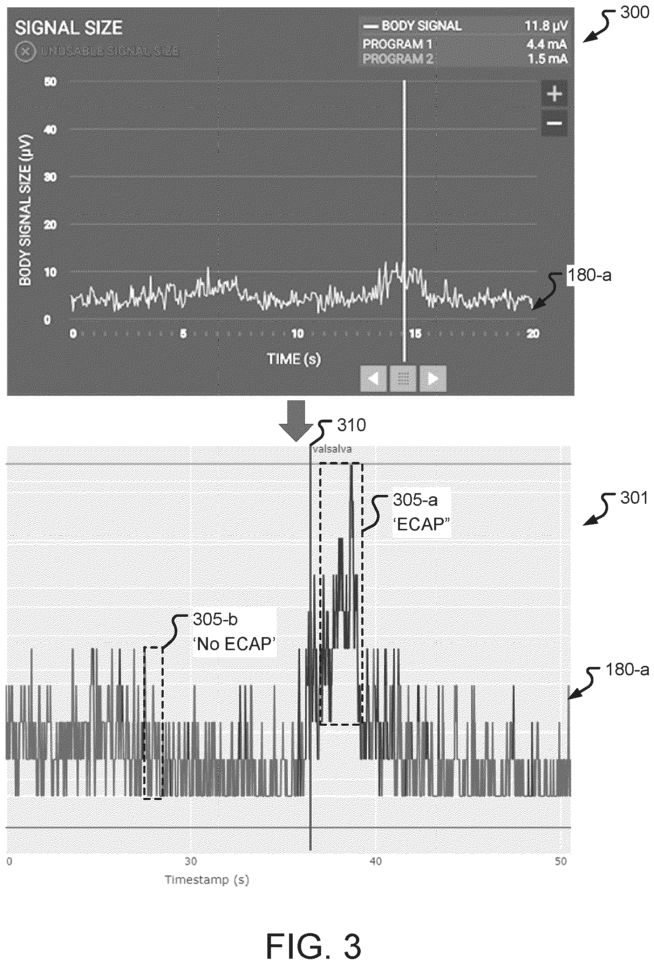

Fig. 3 illustrates example views 300 and 301 of a response waveform 180-a corresponding to a data signal 126 provided by a device (e.g., therapy device 162, wearable device 170) in accordance with example aspects of the present disclosure.

Referring to example view 301, portion 305-a of waveform 180-a may correspond to or describe an ECAP response produced in response to application of a neuromodulation therapy. As an example, the ECAP response may be produced in response to application of SCS. Portion 305-b of waveform 180-a may correspond to or describe a non-response (e.g., `No ECAP'). The system 100 and techniques described herein may support assigning classifications 182 to portions 305 (e.g., portion 305-a, portion 305-b, etc.) of the waveform 180-a based on characteristics of the portions 305.

For example, the system 100 and techniques described herein may support assigning a classification 182-a (e.g., `electrical response,' `ECAP') to portion 305-a of the waveform 180-a based on the characteristic information associated with the portion 305-a. In another example, the system 100 and techniques described herein may support assigning another classification 182-b (e.g., `non-response', `no ECAP') to portion 305-b of the waveform 180-a based on the characteristic information associated with the portion 305-b.

In an example, referring to view 301, temporal instance 310 is associated with an event or test (e.g., Valsalva maneuver, etc.) initiated with respect to the patient 148. As illustrated in the example of Fig. 3, the systems and techniques may support classifying portion 305-a (which occurs after temporal instance 310) as an electrical response.

Based on the classifications 182, the system 100 may provide one or more parameters 184 (e.g., stimulation parameters described herein) and one or more electrode configurations 186 associated with delivering therapy to the patient 148 and/or sensing responses to delivering the therapy. As described herein, the system 100 may support identification of ECAPS through the assignment of the classifications 182. The techniques described herein with respect to the identification of ECAPS may support improved determination (e.g., increased efficiency, increased accuracy, etc.) of usable ECAP and threshold equations implemented in the system 100.

Example aspects of technical building blocks supportive of ECAP classification are described herein. The machine learning models 138 (e.g., classifiers) of the system 100 may, in response to processing an input waveform 180 (e.g., waveform 180-a), be capable of predicting whether the input waveform 180 is an electrical response (e.g., `ECAP'), a non-response (e.g., 'no ECAP'), or noise. That is, for example, the machine learning models 138 are capable of classifying portions (e.g., portion 305-a, portion 305-b, etc.) of the input waveform 180 as an electrical response (e.g., `ECAP'), a non-response (e.g., 'no ECAP'), or noise.

In an example, the system 100 may provide a data signal 126 (or portion of the data signal 126) to a machine learning model 138 (e.g., a classifier). The machine learning model 138 may provide an output including classifications 182 corresponding to one or more portions (e.g., portion 305-a, portion 305-b) of the input waveform 180. In some aspects, the output may include parameters 184 (e.g., stimulation parameters described herein) and one or more electrode configurations 186.

Based on the classifications 182 and associated parameters 184 and/or electrode configurations 186, the system 100 may support reduced complexity associated with ECAPS setup. In an example, based on a given classification 182 (e.g., `ECAP'), the system 100 may automatically set parameters 184 (e.g., levels, for example, detection thresholds) and electrode configurations 186 associated with recording data signals 126 associated with a patient 148. In another example, based on a different classification 182 (e.g., `No ECAP') indicating that no ECAP is identified, the system 100 may recommend changing one or more stimulation parameters or one or more recording parameters. In some other examples, based on another different classification 182 (e.g., `noise') indicating noise above a threshold value, the system 100 may recommend changing one or more stimulation parameters or one or more recording parameters to account for the noise.

Accordingly, for example, the systems and techniques described herein include algorithms leveraging a classifier to recommend parameters 184 (e.g., thresholds), identify appropriate electrode configurations 186, and the like in association with therapy delivery, therapy response monitoring, and therapy optimization.

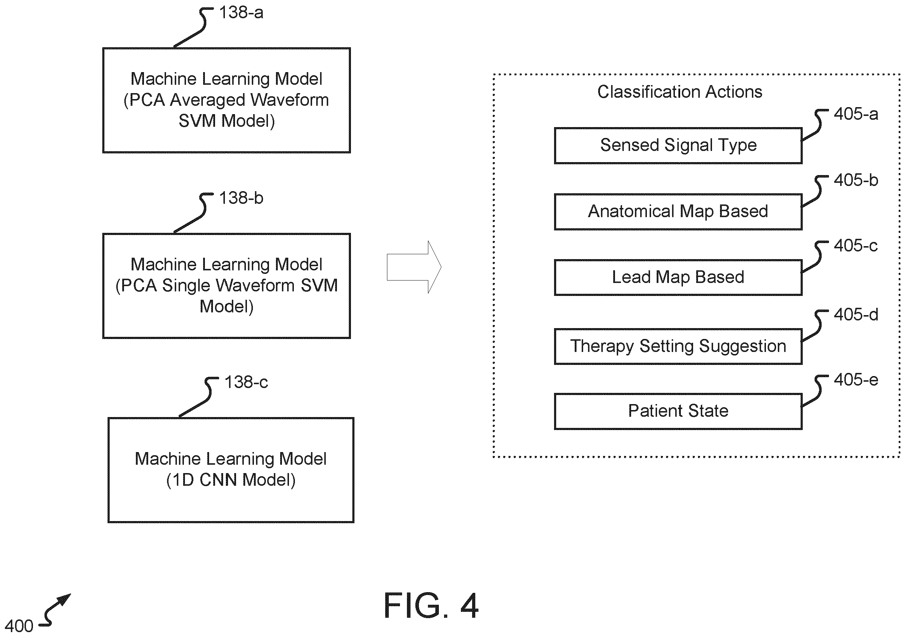

Fig. 4 illustrates an example 400 of machine learning models 138 supported by aspects of the present disclosure.

According to example aspects of the present disclosure, the machine learning models 138 may support classification (e.g., `ECAP' classification, `No ECAP' classification, noise classification, etc.) of raw waveforms associated with a data signal 126. In some other aspects, the example machine learning models 138 may support classification of waveforms generated based on a transform (e.g., a data analysis, principal component analysis (PCA), etc.) applied to a raw waveform. It is to be understood that descriptions of classifying a waveform include classifying portions (e.g., portion 305-a, portion 305-b, etc.) of the waveforms. The machine learning models 138 may be trained based on training data 144 as described herein.

In an example, machine learning model 138-a may be a PCA averaged waveform SVM model. The machine learning model 138-a may support up to about 98.7% accuracy on averaged ECAPs ≥ 4µV. In some aspects, the machine learning model 138-a may support classification using radial basis function (RBF) kernels. The machine learning model 138-a may support identifying ECAPs from averaged data.

In another example, machine learning model 138-b may be a PCA single waveform SVM model. The machine learning model 138-b may support up to about 94.2% accuracy on raw ECAPs ≥ 4µV. In some aspects, classification using machine learning model 138-b and raw waveforms may support quicker response times. In some aspects, noise in waveforms may add some complexity to classification. Accordingly, for example, the machine learning model 138-b may be trained utilizing a PCA transform from averaged waveforms. In an example implementation, the machine learning model 138-b may be trained with two raw waveforms: 273k ('ECAP') and 409k (`No ECAP').

In another example, machine learning model 138-c may be a one-dimensional convolutional neural network model (1D CNN model). The machine learning model 138-c may support up to about 97.3% accuracy on raw ECAPs ≥ 1µV relative to noise at an ECAP threshold (e.g., absolute ECAP estimation response, mean: 2.0µV; 95^{th} Percentile: 3.19µV). In some aspects, classification using machine learning model 138-c and raw waveforms may support quicker response times. In an example implementation, the machine learning model 138-c may support implementations on a frontend encoder-decoder model to identify abnormal waveforms (e.g., unusual artifacts or noise). In an example implementation, the machine learning model 138-c may be trained with two raw waveforms: 208k ('ECAP') and 1 million ('no-ECAP').

According to example aspects of the present disclosure, the models 138 may support a neural network architecture capable of multiple classification techniques. In an example aspect, the neural network architecture may include machine learning model 138-c (e.g., 1D CNN model) followed by a dense layer, in which the dense layer may be used for classifying portions of a waveform based on output from convolutional layers of the machine learning model 138-c. The neural network architecture may include an exponential activation function, as the exponential activation function may support mimicking stimulation artifacts.

The neural network architecture may include a frontend encoder-decoder model to identify abnormal inputs (e.g., noise, unusual artifacts). The neural network architecture may include an element-wise multiplication layer, particularly when paired with frequency domain transformation of an input data signal 126 (e.g., ECAP input signal). For example, frequency analysis may downplay ECAP latency as a dominating feature with respect to the classification techniques described herein.

The neural network architecture may support K-means clustering using templates (e.g., ECAP templates, etc.) associated with a reference evoked response. In some example implementations, the neural network architecture may support classifying portions of a waveform based on an error value between a recorded input (e.g., data signal 126, waveform corresponding to data signal 126) and one or more templates. In some aspects, the techniques described herein may include utilizing convolution to make time invariant.

The neural network architecture may support transfer of learning. For example, a machine learning model 138 (e.g., machine learning model 138-a, machine learning model 138-b, etc.) may be a model trained through an initial generalized training on reference population data. Aspects of the present disclosure support pairing the machine learning model 138 with a patient 148 (or patient type, patient characteristics, etc.) with respect to therapy delivery and therapy optimization. The systems and techniques may support refining and retraining of the machine learning models 138 such that the models are tailored to the patient 148 (or patient type, patient characteristics, etc.).

The neural network architecture may support vector machines. For example, with reference to machine learning model 138-a (PCA averaged waveform SVM model) and machine learning model 138-b (PCA single waveform SVM model), the neural network architecture may support utilizing the machine learning model 138-a and/or machine learning model 138-b following dimensionality reduction (e.g. PCA or independent component analysis). In an example, with reference to machine learning model 138-a, the neural network architecture may utilize a radial basis function (RBF) kernel following principal component analysis (PCA). In some aspects, the neural network architecture may support the use of time-frequency kernels (e.g., discrete short-time Fourier transform, discrete Wigner-Ville frequency distribution, etc.).

Aspects of the neural network architecture support feeding multiple signal types into the machine learning models 138. For example, in addition to the machine learning models 138 providing classifications 182 and parameters 184 (e.g., stimulation parameters, etc.) based on ECAP sensed data as described herein, the machine learning models 138 may determine the classifications 182 and parameters 184 based on additional data (e.g., included in data 125-a and/or data 125-b) fed to the machine learning models 138. Non-limiting examples of the additional data include temporal information (e.g., date and time), accelerometer data (e.g., associated with therapy device 162, electrodes 166, etc.), impedance data (e.g., corresponding to sensors, electrodes 166, etc.), physiological data (e.g., measured heart rate, measured ECG, etc.), and recharge interval estimation (e.g., associated with therapy device 162), but are not limited thereto.

According to example aspects of the present disclosure, the systems and techniques described herein may support classification actions 405 associated with assigning a classification 182 to a data signal 126 (or to a portion of a waveform 180 associated with the data signal 126). In an example, each classification 182 may include an indication of a sensed signal type, be anatomical map based, be lead map based, include a therapy setting suggestion, include an indication of a patient state, or any appropriate combination thereof.

### Sensed Signal Type - Classification Action 405-a

In an example of a classification 182 indicating sensed signal type, computing device 102 may assign a classification 182-a (e.g., `ECAP'), a classification 182-b (e.g., `No ECAP'), or a classification 182-c (e.g., 'Noise') to a data signal 126 (or to a portion of a waveform 180 associated with the data signal 126). In some aspects, computing device 102 may further categorize noise as electromyographic (EMG) noise, electrical noise, mechanical noise, or the like. For example, in the case of a data signal 126 (or to a portion of a waveform 180 associated with the data signal 126) classified as `Noise,', computing device 102 may assign a further classification 182 (or sub-classification) associated with the type (e.g., EMG, electrical, mechanical, etc.) of noise. In some example aspects, computing device 102 may assign a classification 182-c (e.g., 'Noise') for cases involving a saturation of all or some samples of a sensing signal (e.g., data signal 126). For example, computing device 102 may assign a classification 182-c (e.g., 'Noise') for cases in which one or more samples of data signal 126 approaches or exceeds a saturation threshold.

### Anatomical Map Based - Classification Action 405-b

In an example of a classification 182 that is anatomical map based, computing device 102 may assign a classification 182 for a "location" mode during lead implant. During an operation of inserting the lead at an insertion location, the system 100 may utilize the classification 182 to determine whether an ECAP is observed at the insertion location. Based on an analysis of the ECAP morphology, the system 100 may identify and indicate whether the neural elements targeted by the lead implant and stimulation are stimulated and that no ECMAP is evoked. In addition, for example,, through the classification 182, the computing device 102 may indicate to a clinician if observing ECAP at a location and indicate whether an anatomical element associated with the observed ECAP is a candidate for ERS.

In another example, computing device 102 may assign a classification 182 associated with detected lead migration. For example, the classification 182 may indicate whether lead migration associated with an implanted medical device has occurred. In another example of a classification 182 that is anatomical map based, computing device 102 may assign a classification 182 indicating anatomical information (e.g., spinal level, lead laterality, nerve root, etc.) associated with an electrical response of the patient 148.

### Lead Map Based - Classification Action 405-c

In an example of a classification 182 that is lead map based, computing device 102 may support the identification of stimulation contacts and recording contacts (e.g., electrodes 166) for yielding ECAPs. For example, the computing device 102 may support quickly identify promising stimulation contacts and recording contacts that yield ECAPs. In an example implementation, the techniques may include holding a stimulation contact constant and looping through recording contacts to find informative configurations. For example, for stimulation delivered by a given stimulation contact(s) (e.g., an electrode 166), the techniques described herein may support iterative analysis of data signals recorded by different recording contacts (e.g., other electrodes 166) to identify one or more configurations of stimulation contacts and recording contacts for yielding ECAPs. In some aspects, the configurations may include placement information and quantity associated with the stimulation contacts and recording contacts.

In some examples of a classification 182 that is lead map based, computing device 102 may support indicating whether a lead 168 is stable/encapsulated. For example, a response to neuromodulation therapy may be measured using a lead 168 described herein, and computing device 102 may provide a classification 182 indicating whether the lead 168 is stable, encapsulated, or the like.

### Therapy Setting Suggestion - Classification Action 405-d

In an example of a classification 182 indicating a therapy setting suggestion, computing device 102 may provide a classification 182 indicating recording/stimulation electrodes, stimulation parameters (e.g., stimulation frequency, amplitude, duration, duty cycle, post-stimulus recharge parameters, etc.), and ECAP responsive stimulation threshold levels. Accordingly, for example, by providing a classification 182 (classification result) including therapy setting suggestions associated with the classification 182, aspects of the present disclosure may obviate the need for ECAP responsive stimulation thresholds. The stimulation parameters described herein may include active stimulation parameters, passive stimulation parameters, or any combination thereof in association with a therapy device 162. The stimulation parameters may include customized settings associated with charging or recharging the therapy device 162. Non-limiting examples of the customized settings include custom durations of passive recharge, custom amplitudes and pulse width of active recharge, other appropriate settings associated with recharge, and the like.

In some aspects, the classification 182 may include an indication of optimized therapy settings and recharge interval settings, which may support optimizing therapy settings and recharge interval simultaneously. In an example implementation, the classification 182 may include a prediction of follow up visits by patient 148 with respect to a therapy issue. For example, the classification 182 may include a prediction that a patient 148 will be back within a temporal duration (e.g., in three weeks) due to consistently fast battery consumption associated with therapy device 162. In some aspects, such predictions associated with patient follow up visits/battery consumption associated with therapy device 162 may support reducing clinical-rep burden.

In some cases, a therapy device 162 may be powered by a non-rechargeable primary cell battery. In some alternative and/or additional cases, a therapy device 162 may be powered by a rechargeable cell battery. Accordingly, for example, the aspects described herein associated with recharge interval settings may be applied to recharging a rechargeable cell battery of a therapy device 162.

### Patient State - Classification Action 405-e

In an example of a classification 182 indicating a patient state, computing device 102 may provide a classification 182 indicating whether patient 148 is in a sleep state, an awake state, an active state, a resting state, and the like. In some aspects, computing device 102 may classify aggressor events based on short term ECAP transitions (e.g., awake active state may be associated with rapid transitions in the ECAP signals associated with aggressors, while a sleep state may be indicated with fewer less profound aggressors).

The techniques described herein may support recurrent neural networks (RNN, LSTM, etc.) to classify patient state based on a time series of ECAP waveforms. In an example, the system and techniques described herein may support training a neural network (e.g., one or more machine learning models 138) to detect a patient state (e.g., a sleep state, an awake state, an active state, a resting state, etc.) based on a single ECAP waveform. In another example, the system and techniques described herein may support training the neural network to detect a patient state (and/or characteristics associated with the patient state) based on a time series of ECAP waveforms.

In another example, the techniques described herein may support classification actions associated with medication (e.g., change in ECAP morphology/timing). For example, the techniques described herein may include assigning a classification 182 to a change in ECAP morphology/timing, and the classification 182 may include an indication of medication information (e.g., medication type, medication dosage, etc.) associated with the change in ECAP morphology/timing.

In some other examples, the techniques described herein may support classification actions associated with indicating whether the patient 148 is in a pain state (e.g., patient increases in stimulation amplitude may correlate with certain ECAP morphology). For example, the techniques described herein may include assigning a classification 182 to a certain ECAP morphology, the classification 182 may include an indication of a pain state associated with the ECAP morphology, and based on the pain state (and the ECAP morphology), the computing device 102 may provide a recommended stimulation amplitude.

In another example, the techniques described herein may support classification actions associated with indicating a therapeutic impact on the patient 148. In an example, the therapeutic impact may include altering (e.g., reducing) a pain state of the patient 148.

In another example, the techniques described herein may support classification actions associated with indicating a position (e.g., standing, sitting, laying down, etc.) of the patient 148. Accordingly, for example, the techniques described herein may support the determination of positionally responsive stimulation, which may thereby support relatively easier setup compared to other techniques. For example, classification actions associated with indicating a position (e.g., standing, sitting, laying down, etc.) of the patient 148 may support reduced complexity associated with setting up therapy delivery and monitoring.

### Population Model Use

According to example aspects of the present disclosure, the systems and techniques described herein may support population model use. For example, the system 100 may support aggregating data (e.g., ECAP data) to a database (e.g., database 130, a cloud database, etc.) for classification of individual patients or other user trends using AI/ML techniques described herein and/or other appropriate AI/ML techniques.

In an example, the system 100 may support aggregating data (e.g., ECAP data) according to patient type (e.g., low responder, medium responder, high responder), together with other appropriate information or settings (e.g., therapy settings, patient visit history/frequency, etc.).

In another example, the system 100 may support aggregating data (e.g., ECAP data) according to patient pain level (e.g., low pain level/medium pain level/high pain level, whether the patient 148 is at rest/active, etc.) as currently self-reported by the patient or automatically determined by the system 100, together with other appropriate information or settings (e.g., pain ratings, accelerometer data associated with therapy device 162 or wearable device 170, etc.)

In some other examples, the system 100 may support aggregating data (e.g., ECAP data) according to battery drain burden of the therapy device 162, together with other appropriate information or settings (e.g., therapy settings associated with the therapy device 162).

Accordingly, for example, the system 100 may support training machine learning models 138 based on the aggregated data (e.g., aggregated ECAP data, therapy settings, device data, patient data, etc.). Using the machine learning models 138, the system 100 may process a data signal 126 (or corresponding waveform 180) associated with a patient 148 and provide a classification 182 indicating predicted patient type associated with the patient 148, predicted pain profile information associated with the patient 148, predicted device performance (e.g., of a therapy device 162) associated with delivering therapy to the patient 148, or an appropriate combination thereof.

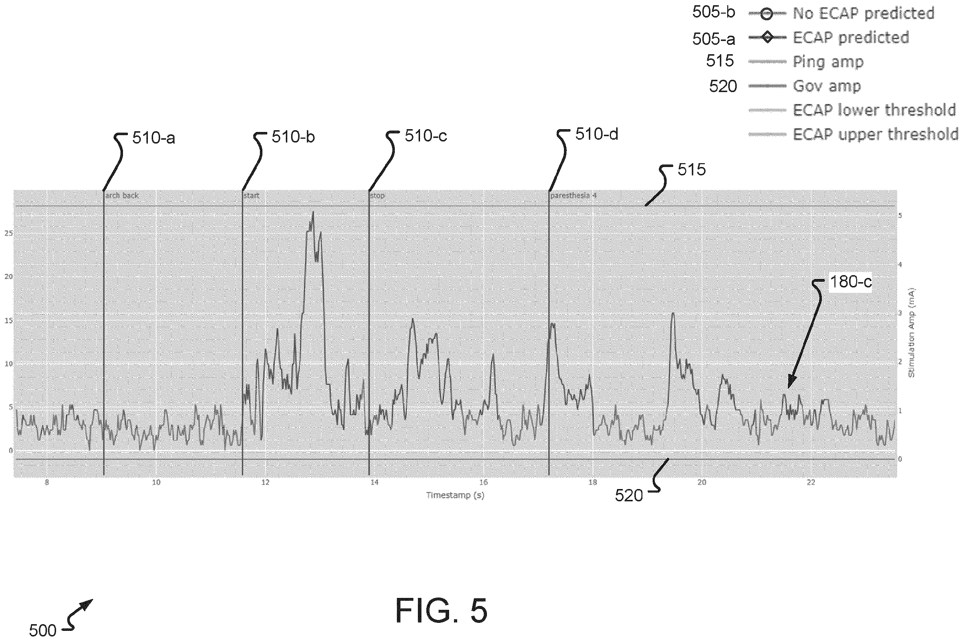

Fig. 5 illustrates an example view 500 of a response waveform 180-c corresponding to a data signal 126 provided by a device (e.g., therapy device 162, wearable device 170) in accordance with aspects of the present disclosure. Fig. 5 further illustrates example classifications 182 assigned to portions 505 of the waveform 180-c.

The system 100 and techniques described herein may support assigning a classification 182-a (e.g., `ECAP predicted') (also referred to herein as an `electrical response' or `ECAP') to portions 505-a of the waveform 180-a based on the characteristic information associated with the portions 505-a. The system 100 and techniques described herein may support assigning classification 182-b (e.g., `No ECAP predicted') (also referred to herein as `No ECAP') to portions 505-b of the waveform 180-c based on the characteristic information associated with the portions 505-b.

As described with reference to Fig. 5, aspects of the present disclosure support ECAP classification during movement of a patient 148. In the example of Fig. 5, temporal instance 510-a is associated with a movement (e.g., arching back) of the patient 148. Temporal instance 510-b is associated with initiating delivery of therapy (e.g., electrical stimulation, an electrical pulse, etc.) to the patient 148. Temporal instance 510-c is associated with pausing or stopping delivery of the therapy. Temporal instance 510-d is associated with the start of a physiological response (e.g., paresthesia) of the patient 148 related to the therapy. Fig. 5 illustrates examples of a ping amplitude 515 and a gov amplitude 520.

As illustrated with reference to Fig. 5, the system 100 and techniques described herein support providing ECAP detection without windows or thresholds. That is, for example, the trained machine learning models 138 described herein may support recognition of waveform patterns or waveform characteristics associated with an `ECAP response,' `No ECAP response,' 'noise', and the like as described herein.

The system 100 and techniques described herein support assigning classifications 182 to portions 505 (e.g., portions 505-a, portions 505-b, etc.) of the waveform 180-c, absent using a temporal window associated with detecting or sensing the data signal 126 corresponding to the waveform 180-c. That is, for example, the techniques described herein support ECAP detection without implementing a target sensing window (e.g., a temporal window after a delivering a stimulus) for ECAP detection.

In another example, the system 100 and techniques described herein support assigning classifications to portions 505 (e.g., portions 505-a, portions 505-b, etc.) of the waveform 180-c, absent using a threshold value based on which to assign the classifications 182. That is, for example, the techniques described herein support ECAP detection without implementing threshold values. For example, the techniques described herein support ECAP detection without reliance on a threshold ECAP amplitude (e.g., measured as the amplitude between a first negative peak (N1) and a second positive peak (P2)), an ECAP lower threshold value, an ECAP upper threshold value, and the like for ECAP detection.

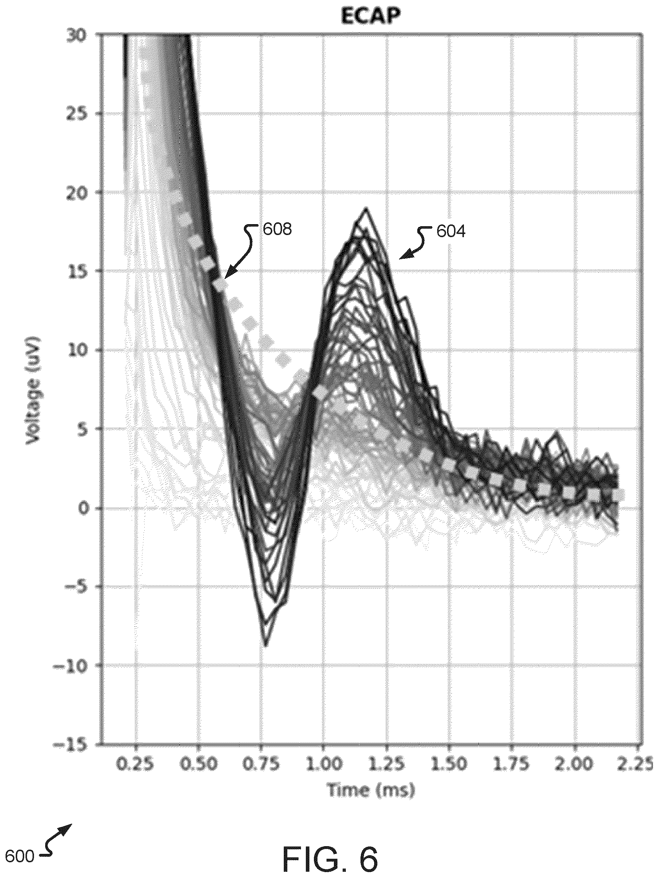

Fig. 6 is an example view 600 of response waveforms 604 corresponding to data signals 126 provided by a device (e.g., therapy device 162, wearable device 170) in accordance with aspects of the present disclosure. In some embodiments, response waveforms 604 may correspond to or describe ECAP responses produced in response to application of respective therapies delivered to a patient 148. As an example, the ECAP responses may be produced in response to application of SCS (e.g., according to different stimulation parameters). Fig. 6 provides an example of recorded ECAPs and the stimulation artifact exponential decay. In some aspects of the present disclosure, the systems and techniques may support developing ECAP templates (e.g., for ECAP detection described herein) from averaging real data of response waveforms 604.

Plot 608 is an example of the artifact model that may be fit to the raw ECAP recording data acquired from the device (e.g., fit to response waveforms 604). In some embodiments, the techniques described herein include deriving the artifact model through fitting the closest artifact model to the observed data. In some examples, the techniques described herein may include subtracting the artifact model from the ECAP data, thereby producing a cleaned-up data set upon which the system 100 may apply further operations (e.g., classification).

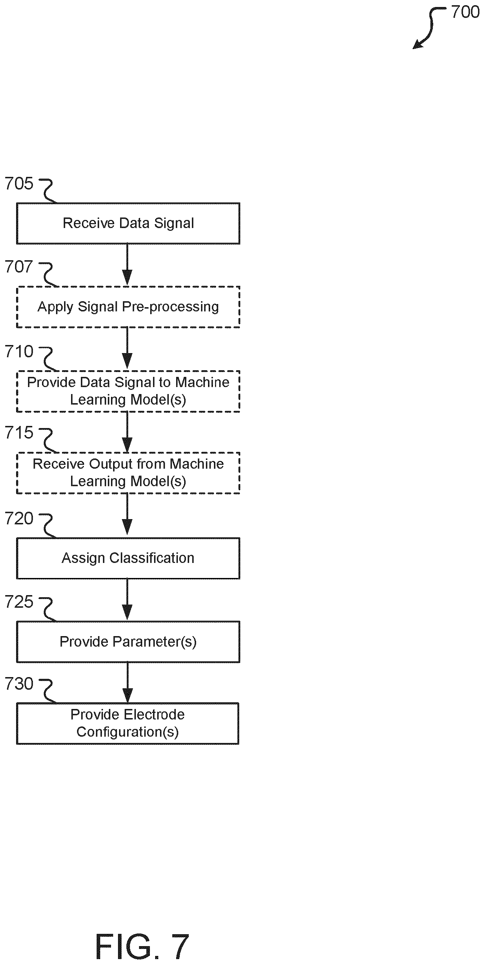

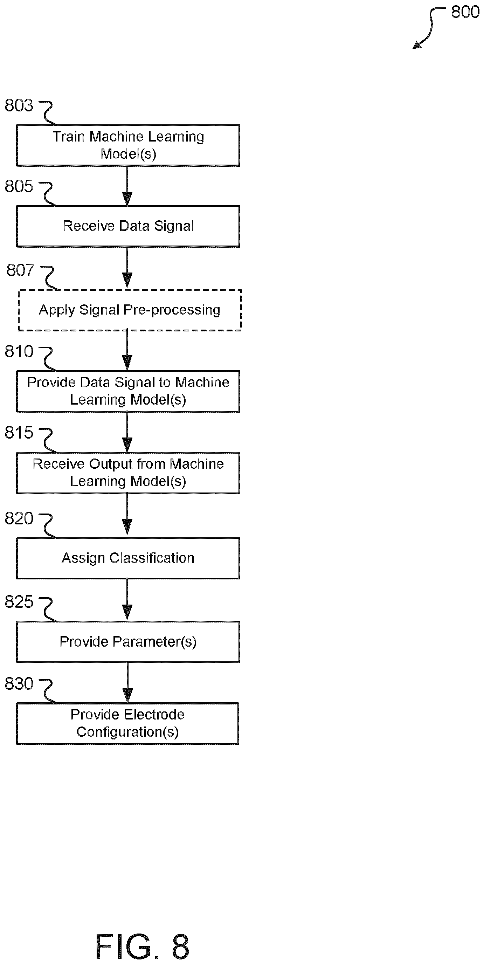

Figs. 7 and 8 illustrate example of process flows in accordance with aspects of the present disclosure. In some examples, the process flows may be implemented by aspects of system 100 (e.g., computing device 102, system 160, etc.) described with reference to Figs. 1 and 2.

In the following descriptions of the process flows, the operations may be performed in a different order than the order shown, or the operations may be performed in different orders or at different times. Certain operations may also be left out of the process flows, or one or more operations may be repeated, or other operations may be added to the process flows.

It is to be understood that any appropriate device (e.g., computing device 102, therapy device 162, wearable device 170, another device in communication with the computing device 102, therapy device 162, or wearable device 170, etc.) may perform the operations shown.

The process flows may be implemented by a system 100 including: a processor (e.g., processor 104) and a memory (e.g., memory 106) storing data thereon that, when processed by the processor, cause the processor to perform one or more operations of the process flows.

The process flows (and/or one or more operations thereof) described herein may be carried out or otherwise performed, for example, by at least one processor. The at least one processor may be the same as or similar to the processor(s) 104 of the computing device 102 described above. The at least one processor may be part of system 160, therapy device 162, or wearable device 170. A processor other than any processor described herein may also be used to execute the process flows. The at least one processor may perform operations of the process flows by executing elements stored in a memory such as the memory 106. The elements stored in memory and executed by the processor may cause the processor to execute one or more operations of a function as shown in the process flows. One or more portions of the process flows may be performed by the processor executing any of the contents of memory.

Referring to Fig. 7, at 705, the process flow 700 may include receiving a data signal from one or more sensors associated with the system in response to therapy delivered to a patient.

In some aspects, the data signal includes an evoked compound action potential (ECAP) signal, an evoked compound muscle action potential (ECMAP) signal, or a combination thereof.

In some aspects, the therapy includes neuromodulation therapy. In some aspects, the therapy includes at least one of: spinal cord stimulation; peripheral nerve stimulation; and pelvic stimulation. In some other aspects, the neuromodulation therapy may include any appropriate therapy (e.g., deep brain stimulation (DBS), delivering stimulation, measuring response at any anatomical element, etc.)

The system further includes a device (e.g., therapy device 162, wearable device 170) to deliver the therapy and receive the data signal from the one or more sensors. In an example, the device includes at least one of a medical device, a wearable device, and an implanted device.

At 720, the process flow 700 may include assigning a classification to one or more portions of a waveform associated with the data signal based on characteristic information associated with the one or more portions of the waveform.

In some aspects, the system further includes a device (e.g., computing device 102, therapy device 162, wearable device 170) that generates the waveform based on the data signal received from the one or more sensors.

In some aspects, the classification is included in a set of classifications including: a first classification indicating the one or more portions of the waveform as an electrical response by one or more anatomical elements of the patient in association with delivering the therapy; a second classification indicating the one or more portions of the waveform as a non-response by the one or more anatomical elements in association with delivering the therapy; and a third classification indicating the one or more portions of the waveform as noise.

In some aspects, the classification indicates the one or more portions of the waveform as a non-response or noise, based on comparing the one or more portions of the waveform to one or more reference artifacts.

In some aspects, the classification indicates the one or more portions of the waveform as an evoked response, based on comparing the one or more portions of the waveform to one or more waveform templates associated with a reference evoked response.

In some aspects, the waveform includes a principal component analysis (PCA) of the waveform generated based on the data signal. In some aspects, the waveform includes a raw waveform corresponding to the data signal.

In some aspects, assigning the classification is further based on at least one of: temporal information associated with the data signal; frequency information associated with the data signal; accelerometer data corresponding to one or more sensors associated with monitoring physiological information associated with the patient; impedance data corresponding to the one or more sensors; and measured values associated with the physiological information.

In some aspects, assigning the classification is absent a temporal window associated with detecting the data signal by the one or more sensors.

In some aspects, assigning the classification is absent a threshold value associated with the waveform.

In some aspects, the classification includes an indication of at least one of: a signal type associated with the data signal; anatomical information associated with the patient and the data signal; mapping information corresponding to the one or more sensors, one or more second sensors associated with delivering the therapy, or both; the one or more parameters associated with delivering the therapy; and state information associated with the patient.

In some aspects, the classification includes an indication of at least one of: predicted patient type associated with the patient; predicted pain profile information associated with the patient; and predicted device performance associated with delivering the therapy.

In some aspects, providing the classification and the one or more parameters described herein may be based on an output provided by one or more machine learning models. For example, at 707, the process flow 700 may include applying signal pre-processing (e.g., PCA, etc.) to the data signal before providing the data signal to the one or more machine learning models. In some other examples, the process flow 700 may refrain from applying the signal pre-processing of 707.

At 710, the process flow 700 may include providing at least a portion of the data signal to one or more machine learning models. At 715, the process flow 700 may include receiving an output from the one or more machine learning models in response to the one or more machine learning models processing at least the portion of the data signal, wherein the output includes the classification and the one or more parameters. In some aspects, the one or more machine learning models include one or more of the following: one or more support vector machines (SVMs); one or more convolutional neural network (CNN) models; one or more feed forward neural network models; one or more transformer neural network models; and one or more decision trees.

At 725, the process flow 700 may include providing, based on the classification, one or more parameters associated with delivering the therapy.

In some aspects, the one or more parameters include one or more stimulation parameters associated with delivering the therapy and evoking a response.

At 730, the process flow 700 may include providing, based on the classification, a first electrode configuration associated with delivering the therapy, a second electrode configuration associated with sensing a response to delivering the therapy, or both.

Referring to Fig. 8, at 803, the process flow 800 may include training a plurality of machine learning models (e.g., machine learning models 138) based on a training data set associated with one or more therapies delivered to a plurality of reference patients, wherein the training data set includes a plurality of reference data signals received from one or more second sensors based on the one or more therapies delivered to the plurality of reference patients.

At 805, the process flow 800 may include receiving a data signal from one or more sensors associated with the system in response to therapy delivered to a patient.

At 807, the process flow 800 may include applying signal pre-processing (e.g., PCA, etc.) to the data signal before providing the data signal to one or more machine learning models. In another example, the process flow 800 may refrain from applying signal pre-processing.

At 810, the process flow 800 may include providing at least a portion of the data signal to the one or more machine learning models.

At 815, the process flow 800 may include receiving an output from the one or more machine learning models in response to the one or more machine learning models processing at least the portion of the data signal, wherein the output includes the classification and the one or more parameters.

At 820, the process flow 800 may include assigning a classification to one or more portions of a waveform associated with the data signal based on characteristic information associated with the one or more portions of the waveform. In some aspects, assigning the classification at 820 is based on at least one machine learning model included in the plurality of machine learning models processing at least the portion of the data signal.

At 825, the process flow 700 may include providing, based on the classification, one or more parameters associated with delivering the therapy

At 830, the process flow 700 may include providing, based on the classification, a first electrode configuration associated with delivering the therapy, a second electrode configuration associated with sensing a response to delivering the therapy, or both.

As noted above, the present disclosure encompasses methods with fewer than all of the steps identified in Figs. 7 and 8 (and the corresponding description of the process flows), as well as methods that include additional steps beyond those identified in Figs. 7 and 8 (and the corresponding description of the process flows). The present disclosure also encompasses methods that include one or more steps from one method described herein, and one or more steps from another method described herein. Any correlation described herein may be or include a registration or any other correlation.

The foregoing is not intended to limit the disclosure to the form or forms disclosed herein. In the foregoing Detailed Description, for example, various features of the disclosure are grouped together in one or more aspects, implementations, and/or configurations for the purpose of streamlining the disclosure. The features of the aspects, implementations, and/or configurations of the disclosure may be combined in alternate aspects, implementations, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, implementation, and/or configuration. Thus, the following claims are hereby incorporated into this Detailed Description, with each claim standing on its own as a separate preferred implementation of the disclosure.

Moreover, though the foregoing has included description of one or more aspects, implementations, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, implementations, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

Example aspects of the present disclosure include:
A system including: a processor; and a memory storing data thereon that, when processed by the processor, cause the processor to: receive a data signal from one or more sensors associated with the system in response to therapy delivered to a patient; assign a classification to one or more portions of a waveform associated with the data signal based on characteristic information associated with the one or more portions of the waveform; and provide, based on the classification, one or more parameters associated with delivering the therapy.

Any of the aspects herein, wherein the classification is included in a set of classifications including: a first classification indicating the one or more portions of the waveform as an electrical response by one or more anatomical elements of the patient in association with delivering the therapy; a second classification indicating the one or more portions of the waveform as a non-response by the one or more anatomical elements in association with delivering the therapy; and a third classification indicating the one or more portions of the waveform as noise.

Any of the aspects herein, wherein the one or more parameters include one or more stimulation parameters associated with delivering the therapy and evoking a response.

Any of the aspects herein, wherein the data is further executable by the processor to: provide, based on the classification, a first electrode configuration associated with delivering the therapy, a second electrode configuration associated with sensing a response to delivering the therapy, or both.

Any of the aspects herein, wherein the data is further executable by the processor to: provide at least a portion of the data signal to one or more machine learning models; and receive an output from the one or more machine learning models in response to the one or more machine learning models processing at least the portion of the data signal, wherein the output includes the classification and the one or more parameters.

Any of the aspects herein, wherein the one or more machine learning models include one or more of the following: one or more support vector machines (SVMs); one or more convolutional neural network (CNN) models; one or more feed forward neural network models; one or more transformer neural network models; and one or more decision trees.

Any of the aspects herein, wherein the waveform includes a principal component analysis (PCA) of the waveform generated based on the data signal.

Any of the aspects herein, wherein the waveform includes a raw waveform corresponding to the data signal.

Any of the aspects herein, wherein the classification indicates the one or more portions of the waveform as a non-response or noise, based on comparing the one or more portions of the waveform to one or more reference artifacts.

Any of the aspects herein, wherein the classification indicates the one or more portions of the waveform as an evoked response, based on comparing the one or more portions of the waveform to one or more waveform templates associated with a reference evoked response.

Any of the aspects herein, wherein the data signal includes an evoked compound action potential (ECAP) signal, an evoked compound muscle action potential (ECMAP) signal, or a combination thereof.

Any of the aspects herein, wherein assigning the classification is further based on at least one of: temporal information associated with the data signal; frequency information associated with the data signal; accelerometer data corresponding to one or more sensors associated with monitoring physiological information associated with the patient; impedance data corresponding to the one or more sensors; and measured values associated with the physiological information.

Any of the aspects herein, wherein assigning the classification is absent a temporal window associated with detecting the data signal by the one or more sensors.

Any of the aspects herein, wherein assigning the classification is absent a threshold value associated with the waveform.

Any of the aspects herein, wherein the classification includes an indication of at least one of: a signal type associated with the data signal; anatomical information associated with the patient and the data signal; mapping information corresponding to the one or more sensors, one or more second sensors associated with delivering the therapy, or both; the one or more parameters associated with delivering the therapy; and state information associated with the patient.

Any of the aspects herein, wherein the classification includes an indication of at least one of: predicted patient type associated with the patient; predicted pain profile information associated with the patient; and predicted device performance associated with delivering the therapy.

Any of the aspects herein, wherein the therapy includes neuromodulation therapy.

Any of the aspects herein, wherein the therapy includes at least one of: spinal cord stimulation; peripheral nerve stimulation and pelvic stimulation.

Any of the aspects herein, wherein the data is further executable by the processor to: train a plurality of machine learning models based on a training data set associated with one or more therapies delivered to a plurality of reference patients, wherein the training data set includes a plurality of reference data signals received from one or more second sensors based on the one or more therapies delivered to the plurality of reference patients, wherein assigning the classification is based on at least one machine learning model included in the plurality of machine learning models processing at least a portion of the data signal.

Any of the aspects herein, further including a device to deliver the therapy and receive the data signal from the one or more sensors.

Any of the aspects herein, wherein the device includes at least one of a medical device, a wearable device, and an implanted device.

Any of the aspects herein, further including: a device that generates the waveform based on the data signal received from the one or more sensors.

A device including: one or more electrodes; a processor; and a memory storing data thereon that, when processed by the processor, cause the processor to: receive a data signal from the one or more electrodes in response to therapy delivered to a patient; assign a classification to one or more portions of a waveform associated with the data signal based on characteristic information associated with the one or more portions of the waveform; and provide, based on the classification, one or more parameters associated with delivering the therapy.

A method including: receiving a data signal from one or more sensors in response to therapy delivered to a patient; assigning a classification to one or more portions of a waveform associated with the data signal based on characteristic information associated with the one or more portions of the waveform; and providing, based on the classification, one or more parameters associated with delivering the therapy.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/implementations in combination with any one or more other aspects/features/implementations.

Use of any one or more of the aspects or features as disclosed herein.

It is to be appreciated that any feature described herein can be claimed in combination with any other feature(s) as described herein, regardless of whether the features come from the same described implementation.

The phrases "at least one," "one or more," "or," and "and/or" are open-ended expressions that are both conjunctive and disjunctive in operation. For example, each of the expressions "at least one of A, B and C," "at least one of A, B, or C," "one or more of A, B, and C," "one or more of A, B, or C," "A, B, and/or C," and "A, B, or C" means A alone, B alone, C alone, A and B together, A and C together, B and C together, or A, B and C together.

The term "a" or "an" entity refers to one or more of that entity. As such, the terms "a" (or "an"), "one or more," and "at least one" can be used interchangeably herein. It is also to be noted that the terms "comprising," "including," and "having" can be used interchangeably.

The term "automatic" and variations thereof, as used herein, refers to any process or operation, which is typically continuous or semi-continuous, done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material."

Aspects of the present disclosure may take the form of an implementation that is entirely hardware, an implementation that is entirely software (including firmware, resident software, micro-code, etc.) or an implementation combining software and hardware aspects that may all generally be referred to herein as a "circuit," "module," or "system." Any combination of one or more computer-readable medium(s) may be utilized. The computer-readable medium may be a computer-readable signal medium or a computer-readable storage medium.

A computer-readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer-readable storage medium may be any tangible medium that can contain or store a program for use by or in connection with an instruction execution system, apparatus, or device.

A computer-readable signal medium may include a propagated data signal with computer-readable program code embodied therein, for example, in baseband or as part of a carrier wave. Such a propagated signal may take any of a variety of forms, including, but not limited to, electro-magnetic, optical, or any suitable combination thereof. A computer-readable signal medium may be any computer-readable medium that is not a computer-readable storage medium and that can communicate, propagate, or transport a program for use by or in connection with an instruction execution system, apparatus, or device. Program code embodied on a computer-readable medium may be transmitted using any appropriate medium, including, but not limited to, wireless, wireline, optical fiber cable, RF, etc., or any suitable combination of the foregoing.

The terms "determine," "calculate," "compute," and variations thereof, as used herein, are used interchangeably and include any type of methodology, process, mathematical operation or technique.

## Claims

1. A system (100) comprising:
a processor (104); and
a memory (106) storing data (125) thereon that, when processed by the processor (104), cause the processor (104) to:
receive a data signal (126) from one or more sensors associated with the system (100) in response to therapy delivered to a patient (148);
assign a classification (182) to one or more portions (305) (505) of a waveform (180) associated with the data signal (126) based on characteristic information associated with the one or more portions (305) (505) of the waveform (180); and
provide, based on the classification (182), one or more parameters (184) associated with delivering the therapy.

2. The system (100) of claim 1, wherein the classification (182) is comprised in a set of classifications comprising:
a first classification (182) indicating the one or more portions (305) (505) of the waveform (180) as an electrical response by one or more anatomical elements of the patient (148) in association with delivering the therapy;
a second classification (182) indicating the one or more portions (305) (505) of the waveform (180) as a non-response by the one or more anatomical elements in association with delivering the therapy; and
a third classification (182) indicating the one or more portions (305) (505) of the waveform (180) as noise.

3. The system (100) of any of claims 1 to 2, wherein the data (125) is further executable by the processor (104) to:
provide, based on the classification (182), a first electrode configuration associated with delivering the therapy, a second electrode configuration associated with sensing a response to delivering the therapy, or both.

4. The system (100) of any of claims 1 to 3, wherein the data (125) is further executable by the processor (104) to:
provide at least a portion (305) of the data signal (126) to one or more machine learning models (138); and
receive an output from the one or more machine learning models (138) in response to the one or more machine learning models (138) processing at least the portion (305) of the data signal (126), wherein the output comprises the classification (182) and the one or more parameters (184).

5. The system (100) of any of claims 1 to 4, wherein the waveform (180) comprises a principal component analysis (PCA) of the waveform (180) generated based on the data signal (126).

6. The system (100) of any of claims 1 to 5, wherein the waveform (180) comprises a raw waveform (180) corresponding to the data signal (126).

7. The system (100) of any of claims 1 to 6, wherein the classification (182):
indicates the one or more portions (305) (505) of the waveform (180) as a non-response or noise, based on comparing the one or more portions (305) (505) of the waveform (180) to one or more reference artifacts; or
indicates the one or more portions (305) (505) of the waveform (180) as an evoked response, based on comparing the one or more portions (305) (505) of the waveform (180) to one or more waveform (180) templates associated with a reference evoked response.

8. The system (100) of any of claims 1 to 7, wherein the data signal (126) comprises an evoked compound action potential (ECAP) signal, an evoked compound muscle action potential (ECMAP) signal, or a combination thereof.

9. The system (100) of any of claims 1 to 8, wherein assigning the classification (182) is further based on at least one of:
temporal information associated with the data signal (126);
frequency information associated with the data signal (126);
accelerometer data (125) corresponding to one or more sensors associated with monitoring physiological information associated with the patient (148);
impedance data (125) corresponding to the one or more sensors; and
measured values associated with the physiological information.

10. The system (100) of any of claims 1 to 9, wherein assigning the classification (182) is absent a temporal window associated with detecting the data signal (126) by the one or more sensors.

11. The system (100) of any of claims 1 to 10, wherein assigning the classification (182) is absent a threshold value associated with the waveform (180).

12. The system (100) of any of claims 1 to 11, wherein the classification (182) comprises an indication of at least one of:
a signal type associated with the data signal (126);
anatomical information associated with the patient (148) and the data signal (126);
mapping information corresponding to the one or more sensors, one or more second sensors associated with delivering the therapy, or both;
the one or more parameters (184) associated with delivering the therapy; and
state information associated with the patient (148).

13. The system (100) of any of claims 1 to 12, wherein the classification (182) comprises an indication of at least one of:
predicted patient (148) type associated with the patient (148);
predicted pain profile information associated with the patient (148); and
predicted device performance associated with delivering the therapy.

14. A device comprising:
one or more electrodes (166);
a processor (104); and
a memory (106) storing data (125) thereon that, when processed by the processor (104), cause the processor (104) to:
receive a data signal (126) from the one or more electrodes (166) in response to therapy delivered to a patient (148);
assign a classification (182) to one or more portions (305) (505) of a waveform (180) associated with the data signal (126) based on characteristic information associated with the one or more portions (305) (505) of the waveform (180); and
provide, based on the classification (182), one or more parameters (184) associated with delivering the therapy.

15. A method comprising:
receiving a data signal (126) from one or more sensors in response to therapy delivered to a patient (148);
assigning a classification (182) to one or more portions (305) (505) of a waveform (180) associated with the data signal (126) based on characteristic information associated with the one or more portions (305) (505) of the waveform (180); and
providing, based on the classification (182), one or more parameters (184) associated with delivering the therapy.
